# EUROPEAN PATENT SPECIFICATION

(11) **EP 0 458 907 B1**
(45) Date of publication and mention of the grant of the patent: **05.10.1994**
(21) Application number: 90904975.1
(22) Date of filing: 12.03.1990
(51) Int. Cl.: A61K 7/16

(54) **A PREPARATION INTENDED TO PREVENT THE EMISSION OF MERCURY FROM AMALGAM FILLINGS**
ZUBEREITUNG ZUR VERHINDERUNG DES QUECKSILBERAUSSTOSSES AUS AMALGAMZAHNFÜLLUNGEN
PREPARATION DESTINEE A EMPECHER L'EMISSION DE MERCURE A PARTIR DE PLOMBAGES D'AMALGAME

(30) Priority: 13.03.1989 SE 8900872
(43) Date of publication of application: 04.12.1991
(73) Proprietor: HULTEN, Hans-Olle, CH-6648 Minusio (CH); JOHNSSON, Eston, S-331 00 Värnamo (SE); WAERN, Roland, S-331 00 Värnamo (SE)
(72) Inventor: JÖNSSON, Eston, S-331 00 Värnamo (SE); WAERN, Roland, S-331 00 Värnamo (SE)
(74) Representative: Wallengren, Yngvar
(86) International application number: PCT/SE90/00157
(87) International publication number: WO 90/10433

(56) References cited:
- EP-A-02 425 53
- SE-A-87 001 574

## Description

This invention relates to a composition containing sulphur in free form for dental uses.

The invention also includes such a composition intended for the prevention or reduction of the emission of mercury or mercury vapour from amalgam fillings in teeth.

### PRIOR ART

Since long a significant amount of restoration in human teeth is made by means of amalgam. The amalgam used is an alloy of mercury and other metals such as silver, tin, copper and zinc. The amount of mercury in these types of amalgam is mainly between 50 - 70% by weight.

Recent studies demonstrate that metallic mercury is distributed from the amalgam as a vapour. This emission of mercury vapour is a continuing ongoing process, but the amount per second demonstrates significant fluctuations depending on several factors such as the temperature of amalgam filling, or if the filling is exposed to abrasion such as chewing, brushing, grinding or other types of mechanical force. Accordingly, several studies show that the emission of mercury vapour demonstrates significantly higher amounts than during periods of no mechanical stress.

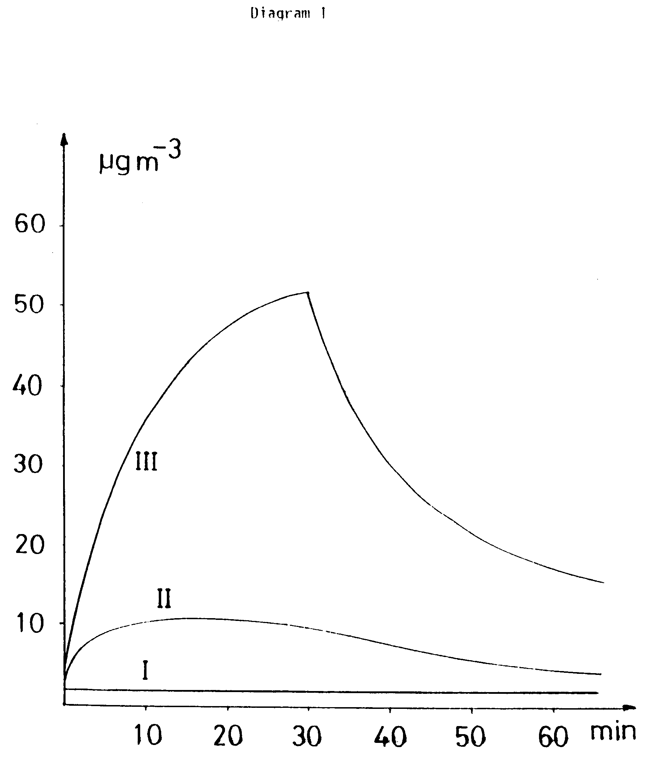

In the publication "Striden om amalgamet", page 21 Källa 33, by the Swedish Federal Research Department report from 1989 measurements are reported of this emission of mercury vapour. Probably these measurements are made on the total exhaled air from test persons and are accordingly demonstrating "diluted" sum numbers from all the teeth in the persons' mouth. Reported results are shown in enclosed diagram 1. In this diagram curve I is showing a series of measurements made on eleven test persons without amalgam fillings. Curve II shows a series of measurements made on nine test persons where each person has up to four amalgam fillings on the occlusal surfaces. Curve III shows a series of measurements made on ten test persons, where each person has more than ten amalgam fillings on their occlusal surfaces. The curves show how the emission of mercury vapour measured in microgram per cubic metre of the exhaled air varies in time when the test persons are chewing.

Curve I shows that also test persons with no amalgam filling in their teeth are emitting a small amount of mercury vapour, where, however, this amount is not influenced by chewing. This can probably be explained in such a way that mercury, deposited in the human body through other ways of supplying than amalgam fillings, continuously is emitted.

From curve II can be seen that the amount of mercury vapour in the exhalation air increases sharply and very quickly when chewing is being started. Further, can be see, that after completed chewing, the amount of mercury vapour after 30 minutes only slowly is falling down to the starting value.

Curve III demonstrates very sharply an increased emisson of mercury vapour and it can be seen that the maximum values after 30 minutes of chewing are many times higher than the starting point before the chewing was initiated. Further can be seen that the increased values remain during a long time after completed chewing. Since the emitted mercury vapour probably at least partly is absorbed by the human body, you cannot eliminate risks.

EP 242 553 discloses topical pharmaceutical compositions for odontostomatology. These, compositions contains, as active principles, allantoin and sulphur. The content of sulphur is 10% or higher.

SE-A-8700157-4 discloses a composition intended for preventing mercury in amalgam fillings from entering the human body and cause poisoning. The composition of this application contains a selenium compound, particularly selenium iodide, as the active agent.

### PRESENTATION OF THE PROBLEM

The purpose of the invention therefore generally is to point out methods by which a reduction of this type of mercury supply to the human body can be made of most considerable proportions. Especially, the purpose of the invention is to provide a composition of the initially mentioned type, which composition has the property to reduce the emission of mercury vapour from amalgam fillings sharply.

### SOLUTION OF THE PROBLEM

According to the invention this can be reached, if the initially mentioned composition is characterized in that the sulphur is present in percentages between 5,0 and 0,005 weight %.

In one embodiment of the composition it also contains, besides sulphur, a grinding or polishing substance.

In a second embodiment the composition is characterized in that it is a tooth paste.

In a third embodiment of the invention the composition is characterized in that it is a chewing gum.

In a further embodiment of the invention this is characterized in that the composition is a liquid in the form of a mouth wash water or mouth spray.

In a further embodiment the composition is characterized in that it is a cooling spray for a dental tool meant for drilling, grinding, polishing or cleaning.

In a further embodiment the composition is characterized in that it is containing a drying, congealing or curing adhesive.

### DESCRIPTION OF THE INVENTION

As a result of practical series of test it has been obvious that sulphur in chemically free form has the property of preventing or reducing the emission of mercury vapour from amalgam fillings when added to the oral cavity and especially to amalgam fillings. The exact chemical or physical processes making this possible, are still unknown, but probably the sulphur together with emitted mercury vapour is forming mercurysulphid, which is extremely difficult to dissolve as well in water as in acid solutions. Consequently the mercury sulphide, if swallowed, is supposed to go through the human body in a chemically unmodified form.

Consequently the invention in its most general form concerns the use of sulphur in order to prevent or at least to reduce the emission of mercury or mercury vapour from amalgam fillings in teeth. In connection with this, the invention also includes the use of sulphur for production of a preparation with this purpose. With use is in this respect understood that sulphur, especially in the forms noted below, is to be mixed into a preparation, which is intended for treatment of amalgam fillings.

Through pilot tests, where teeth with amalgam fillings were polished with a rubber cup using a preparation consisting of a mixture of sulphur and vaseline, the inventors have observed that after such a polishing the emission of mercury vapour is being reduced or disappears to a very great extent. The measurements of the emission of mercury vapour have in this connection been carried out by means of a nozzle close to the treated amalgam surface, from where the air has been sucked out and transported via a tube to a photospectrometer. The sulphur contents, that have been used in this connection, are high, preferably 5 weight % or more.

Further series of tests have been carried out to verify the effects of sulphur also in lower contents. At these tests first of all a "rest point" has been measured, where the emission of mercury vapour has been registrated over a "undisturbed" or untreated tooth by means of a nozzle close to the amalgam surface of the tooth. All the registered values are presented in microgram mercury per cubic metre of air. After measurement of the "rest point" or the initial value the actual tooth has been polished under standardized circumstances with a rubber cap covered with ordinary tooth-paste, commercially sold under the name of ACTA and in a following test covered with the same tooth-paste but with an added ingredient of chemically free sulphur, where the sulphur content amounts to 1 weight % of the tooth-paste.

The results of such test series carried out on one test person (subject of experiment) are shown in table 1. A similar test series on another subject of experiment is shown in table 2. It is worth to notice that the subject of experiment 1 is a dentist and therefore through his profession he is supposed to have been exposed to mercury to a great extent. Furthermore he has a considerable number of amalgam fillings. Consequently, mercury could be found as a "disturbance", emitted partly via the respiratory organs and partly from teeth in the environment. Test person 2 has a comparatively small number of amalgam fillings and he has probably not been exposed to mercury to a considerable extent through other sources.

From the measured values shown in table 1 it can be seen that the amalgam fillings continuously, i.e. at rest, are emitting considerable contents of mercury vapour. After polishing with ACTA tooth-paste with no added ingredients the content of emitted mercury vapour increased drastically, at least by a factor 2 and most highly by a factor 10 - 15. After polishing with ACTA tooth-paste with 1 weight % of sulphur added, the contents of mercury in the sucked (inhaled) air at every tooth decreased considerably. Thereby most of these values are lower than the orginally measured "rest point". It should also be mentioned that the polishing with this tooth-paste with sulphur added, occured immediately upon the measurements of the increased values after polishing with tooth-paste with no ingredient added.

From table 2 it is obvious that the measured values are at about the same or a somewhat lower level than for person 1. Also in this case a considerable increase of the content of mercury vapour was reached in the air, sucked close to the amalgam surface, after polishing with a tooth-paste with no added ingredient. Further the values confirm, after polishing, with tooth-paste with sulphur added that a considerable reduction of the content of mercury vapour can be obtained.

In additon to the above experiments it could also be mentioned that mercury vapour from amalgam fillings might be dangerous for the carrier of such fillings as well as for the dental team who is handling or grinding the amalgam fillings. Thee mercury vapour is consequently an occupational hygienical problem for the dental staff.

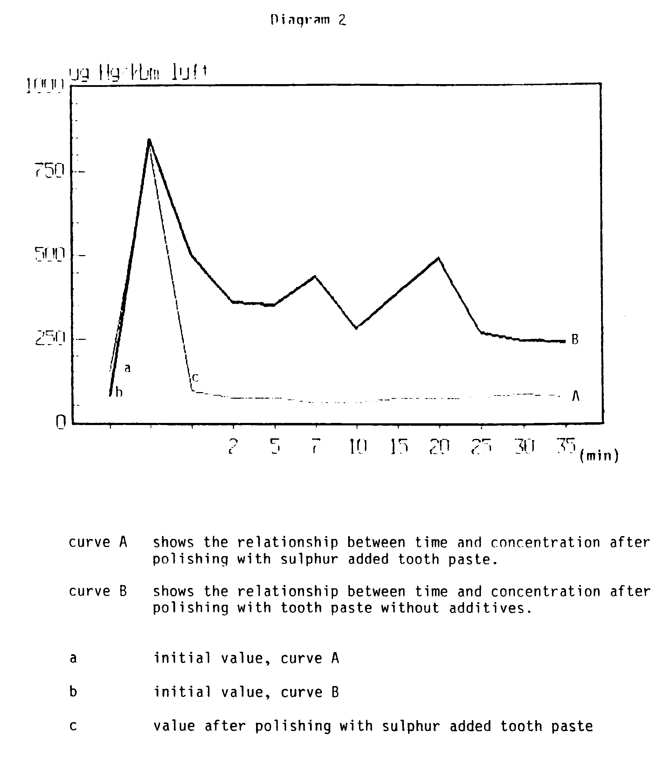

Two series of measurement have been carried out on a third subject of experiment in order to demonstrate the lapses of time of the indicated effects. In the first test series the initial value was determined, whereafter the tooth in question was polished with ACTA tooth-paste with no added ingredient. Measurements were made of the content of mercury vapour in the air close to the polished amalgam surface (tooth 46) and thereafter with intervals as per table 5 and diagram 2.

The decreasing rate amount of the increased value, if no further measures are taken, very clearly corresponds with the result shown in the above mentioned publication "Striden om amalgamet". Consequently, after two minutes, the content of mercury vapour has been considerably reduced from the maximum value immediately upon the polishing and has been further reduced by about 50 % after half an hour. The conclusion of these experiments is that in spite of a rather quick reduction of the content of mercury vapour immediately upon the treatment or stress, there still remains a very increased value during intervals of one hour or more.

The other experimental series (earlier in time than the one mentioned above) on this third test person was carried out in order to make clear how long the duration of the reduction of the content of mercury vapour is, after polishing with a sulphur added tooth-paste. Because of the relative position of the test series, the measurements could not be carried out on the same tooth (tooth 46), which was used for the above experiment of duration, but was made on another tooth (tooth 36), why the figures are not directly comparable.

The results of this second series of measurements are also shown in table 5 and diagram 2.

The conclusion from these test results are that, the obtained reduction of the content of mercury vapour, if sulphur is added, will remain at about the same level for att least half an hour and probably for a longer time.

After that two further experimental series have been carried out on person 3, in order to find out what percentage of sulphur that might be optimal in the tooth paste. Here the measurements have been made in such a way that firstly an initial value was taken and then a value after polishing with a tooth-paste without added ingredient. Hereafter the mouth was washed with water, measures were taken and after that polishing was made with a tooth-paste with sulphur as an added ingredient, followed by mouth wash and measuring. Upon that a renewed polishing was made with a tooth-paste free from added ingredient, mouth wash, measuring and polishing with sulphur added tooth-paste with a different percentage of sulphur, whereafter mouth wash and measuring were carried out, and so on. In this way the following percentages of admixture were tested: 0.1 weight % of sulphur, 0.05 weight % of sulphur, 0.02 weight % of sulphur, 0.01 weight % of sulphur and 0.005 weight % of sulphur. The measurements have been carried out on two different teeth (the teeth 36 and 46) of the test person 3 and the results are being shown in table 3.

From the measured values of table 3 it is possible to make the conclusion that a satisfactory effect is obtained at such low percentage of sulphur as 0.1 weight %. Further there remains a good effect also at such a low percentage as 0.01 weight %. Probably an optimal value is therefore somewhere between these limits and it is also probable that enlarged experimental series would confirm that an optimal value is to be found somewhere between 0.05 and 0.1 weight %.

Since it earlier has been obvious that the favourable influence of the sulphur remains during a rather long time, it might be possible that the figures in table 3 are impaired by some errors, as the measurements have been carried out in a row. This is illustrated by the fact that the measured values, taken immediately upon polishing with a tooth-paste. without sulphur ingredient, have a tendency to decrease, the more experiments that have been made. In order to correct this possible cause of error to some extent, it can be supposed that the measured values after polishing both with a tooth-paste without added ingredient and with tooth-paste with sulphur added, have been influenced to the same degree. By converting the test values in this way, so that the measured figures after polishing with a tooth-paste without added ingredient are supposed to be on the same level, values are to be found as per table 4.

Also from the values shown in table 4 can be seen that the necessary percentages of sulphur to obtain the wanted effect are very low and can be expected to stay under 0.1 weight %.

The sulphur that has been used in all the pilot tests has been of normal trade quality. When measuring the size of grains in a microscope it has been noted that most of the grains have a size of about 0.025 mm. However, both bigger and smaller particles have been observed, why 0.05 - 0.001 mm are probable limits of the size of the grains.

The favourable effects of the admixture of sulphur have been confirmed by practical tests with sulphur added to tooth paste of completely conventional type. However, there is no reason to believe that the favourable effect should depend on the way in which the sulphur is supplied. At least it seems to be clear that the effects are not deteriorated if supply is given in such a way that the sulphur is brought into close contact with the amalgam surfaces. It has also been shown that a favourable effect is obtained with a sulphurous chewing-gum. The invention, however, also is intended to cover a supply through liquid preparations in the form of mouthwash water and mouthspray, which among other things can be used in order to decrease the emission of mercury vapour in dental treatment.

Especially it is worth to point out that the dental staff in its daily work it supposed to be more exposed to mercury vapour to a greater degree than what concerns the average carrier of amalgam fillings. Therefore the invention foresees that dental preparations used by the dental staff have the actual sulphur ingredient added, especially preparations containing grinding or polishing agents.

Further the invention foresees an admixture of sulphur in a preparation base containing a congealing or curing binding agent or adhesive so that the preparation can be brought to the actual tooth surfaces in the form of a congealing, curing or drying varnish which preferably is brought to the amalgam fillings before they are treated. In this case the content of sulphur should be calculated on the dry matter of the actual preparation so that, at the calculation of the content, emitted solvent, if any, is being deducted. Further the invention foresees that the cooling medium (spray), which is used for cooling dental equipment and instruments, has an added ingredient of sulphur.

When the preparation according to the invention appears in a liquid form with a low viscosity, the sulphur is sedimenting comparatively rapidly with the grain sizes mentioned above (0.05 - 0.001 mm), why such a preparation already after a short time of storage has to be suspended or mixed once more before it is possible to use it. The invention therefore foresees the use of smaller particles in these cases, even particles in the colloidal field, i.e. 0.001 -0.000001 mm. Sulphur in colloidal form can of course also be used in other presented methods, where the increased proportion between the surface of the sulphur and its mass can be expected to raise its activity and thereby strengthen its effect.

As can be seen from above the invention also includes a procedure of reducing or preventing the emission of mercury or mercury vapour from amalgam fillings in teeth. In its most elementary way this procedure concerns the supplying of sulphur to the amalgam filling and that it is brought into a close contact with the fillings. As to the invention the sulphur is supplied through a carrier, which has a further purpose, for instance grinding, polishing and reduction of bacterial growth or caries. The sulphur can be mixed in the above mentioned form and in the above mentioned percentages. The method also includes that the sulphur is brought into a very close contact with the amalgam.

**Table 1**

| Test person I | ug Hg/cu.m. air measured close to the tooth: | | | |
|---|---|---|---|---|
| | 14 | 26 | 36 | 46 |
| Before treatment | 90 | 35 | 80 | 85 |
| After polishing with Acta tooth-paste without added ingredient | 215 | 180 | 850 | 1400 |
| After polishing with Acta tooth-paste with 1 weigt % sulphur | 50 | 45 | 52 | 95 |

**Table 2**

| Test person II | ug Hg/cu.m. air measured close to the tooth: | | | |
|---|---|---|---|---|
| | 14 | 26 | 36 | 46 |
| Before treatment | 72 | 45 | 90 | 62 |
| After polishing with Acta tooth-paste without added ingredient | 510 | 660 | 235 | 220 |
| After polishing with Acta tooth-paste with 1 weigt % sulphur | 60 | 86 | 120 | 58 |

Polishing with Acta toothpaste without resp. with sulphur added.

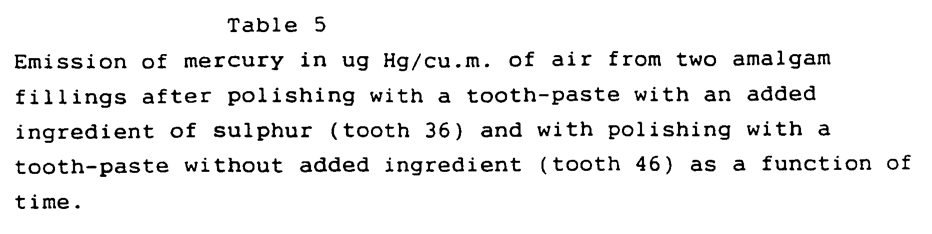

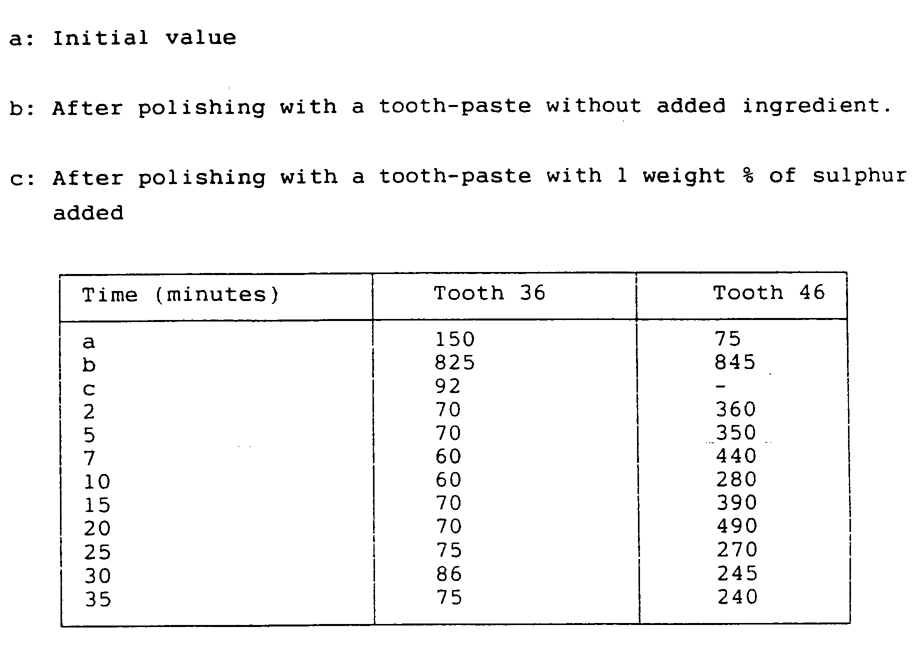

## Claims

1. A composition containing sulphur in free form for dental uses, **characterized** in that the sulphur is present in percentages between 5.0 and 0.005 weight %.

2. Composition as per claim 1, **characterized** in that the sulphur is present in percentages between 1.0 and 0.01 weight %.

3. Composition as per claim 1 or 2, **characterized** in that the sulphur is present in percentages between 0.1 and 0.01 weight %.

4. Composition as per any of the claims 1-3, **characterized** in that the sulphur is present in particle form with a mean size of the particles between 0.05 and 0.000001 mm.

5. Composition as per claim 4, **characterized** in that the sulphur is present in particle form with a mean size of the particles between 0.05 and 0.001 mm.

6. Composition as per claim 4 or 5, **characterized** in that the sulphur is present in particle form with a mean size of the particles between 0.05 and 0.01 mm.

7. Composition as per claim 4, **characterized** in that the sulphur is present in colloidal form.

8. Composition as per any of the claims 1-7, **characterized** in that, besides sulphur, it also contains a grinding or polishing agent.

9. Composition as per any of the claims 1-8, **characterized** in that it is a tooth-paste.

10. Composition as per any of the claims 1-8, **characterized** in that it is a chewing-gum.

11. Composition as per any of the claims 1-8, **characterized** in that it is a liquid in the form of a mouthwash water or mouthspray.

12. Composition as per any of the claims 1-8, **characterized** in that it is a cooling spray for a dental tool meant for drilling, grinding, polishing or cleaning.

13. Composition as per any of the claims 1-8, **characterized** in that it is containing a drying, congealing or curing adhesive.

14. Dental composition as claimed in claim 1 for the prevention or reduction of the emission of mercury and mercury vapour from amalgam fillings in teeth.

## Patentansprüche

1. Eine Zusammensetzung die Schwefel in freier Form beinhaltet, für den dentalen Anwendungsbereich, **gekennzeichnet** dadurch, daß der Schwefel in Gehalte zwischen 5,0 und 0,005 Gesichts% vorkommt.

2. Eine Zusammensetzung gemäß Anspruch 1, **gekennzeichnet** dadurch, daß der Schwefel in Gehalte zwischen 1,0 und 0,01 Gewichts% vorkommt.

3. Eine Zusammensetzung gemäß Anspruch 1 oder 2, **gekennzeichnet** dadurch, daß der Schwefel in Gehalte zwischen 0,1 und 0,01 Gewichts% vorkommt.

4. Eine Zusammensetzung gemäß eines der Ansprüche 1 - 3, **gekennzeichnet** dadurch, daß der Schwefel in Partikelform mit einer Durchschnittspartikelgröße im Bereich von 0,05 - 0,000 001 mm vorkommt.

5. Eine Zusammensetzung gemäß Anspruch 4, **gekennzeichnet** dadurch, daß der Schwefel in Partikelform mit einer Durchschnittspartikelgröße in dem Bereich von 0,05 - 0,001 mm vorkommt.

6. Eine Zusammensetzung gemäß Anspruch 4 oder 5, **gekennzeichnet** dadurch, daß der Schwefel in Partikelform mit einer Durchschnittspartikelgröße in dem Bereich von 0,05 - 0,01 mm vorkommt.

7. Eine Zusammensetzung gemäß Anspruch 4, **gekennzeichnet** dadurch, daß der Schwefel in kolloider Form vorkommt.

8. Eine Zusammentzung gemäß eines der Ansprüche 1 - 7, **gekennzeichnet** dadurch, daß es außer Schwefel auch ein Schleif- oder Poliermittel beinhaltet.

9. Eine Zusammensetzung gemäß eines der Ansprüche 1 - 8. **gekennzeichnet** dadurch, daß es eine Zahnpaste ist.

10. Eine Zusammensetzung gemäß eines der Ansprüche 1 - 8, **gekennzeichnet** dadurch, daß es ein Kaugummi ist.

11. Eine Zusammensetzung gemäß eines der Ansprüche 1 - 8, **gekennzeichnet** dadurch, daß es flüssig in Form eines Mundspülwassers oder Mundspray ist.

12. Eine Zusammensetzung gemäß eines der Ansprüche 1 - 8, **gekennzeichnet** dadurch, daß es eine Kühlflüssigkeit eines Zahnarztwerkzeuges, abgesehen für das Bohren, Schleifen, Putzen, Polieren oder Säubern, ist.

13. Eine Zusammensetzung gemäß eines der Ansprüche 1 - 8, **gekennzeichnet** dadurch, daß es ein trocknendes, erstarrendes oder härtendes Binde- oder Adhäsivmittel ist.

14. Eine dentale Zusammensetzung gemäß Anspruch 1, dafür abgesehen, die Abgabe von Quecksilber und Quecksilberdämpfe von Amalgamfüllungen in Zähnen zu verhindern oder zu reduzieren.

## Revendications

1. Une composition contenant du soufre sous forme chimiquement libre pour l'utilisation dentaire, caracterisée par ce que le soufre est présent en pourcentages entre 5,0 et 0,005% de poids.

2. Composition comme demande 1, caracterisée par ce que le soufre est présent en pourcentage entre 1,0 et 0,01 % de poids.

3. Composition comme demande 1 ou 2, caracterisée par ce que le soufre est présent en pourcentages entre 0,1 et 0,01 % de poids.

4. Composition comme quelqu'une des demandes 1 - 3, caracterisée par ce que le soufre est present sous forme de particule avec une grandeur moyenne des particules entre 0,05 et 0,000001 mm.

5. Composition comme demande 4, caracterisée par ce que le soufre est présent sous forme de particule avec une grandeur moyenne des particules entre 0,05 et 0,001 mm.

6. Composition comme demande 4 ou 5, caracterisée par ce que le soufre est présent sous forme de particule avec une grandeur moyenne des particules entre 0,05 et 0,01 mm.

7. Composition comme demande 4, caracterisée par ce que le soufre est présent sous forme colloïdale.

8. Composition comme quelqu'une des demandes 1 - 7, caracterisée par ce qu'elle, en outre du soufre, aussi contient un agent égrisant ou polissant.

9. Composition comme quelqu'une des demandes 1 - 8, caracterisée par ce qu'elle est un dentifrice.

10. Composition comme quelqu'une des demandes 1 - 8, caracterisée par ce qu'elle est un chewing-gum.

11. Composition comme quelqu'une des demandes 1 - 8, caracterisée par ce qu'elle est un liquide sous forme de l'eau dentifrice ou du spray dentifrice.

12. Composition comme quelqu'une des demandes 1 - 8, caracterisée par ce qu'elle est un spray refroidissant pour un instrument dentaire destiné à fraiser, égriser, polir ou nettoyer.

13. Composition comme quelqu'une des demandes, 1 - 8 caracterisée par ce qu'elle contient un adhésif dessicatif, figeant (congelant) ou polymérisant.

14. Composition dentaire comme demandée dans la demande 1 pour prévention ou réduction de l'émission du mercure ou de la vapeur de mercure des obturations dentaires à l'amalgame.
